# EUROPÄISCHE PATENTSCHRIFT

(11) **EP 0 656 774 B1**
(45) Veröffentlichungstag und Bekanntmachung des Hinweises auf die Patenterteilung: **22.01.1997**
(21) Anmeldenummer: 93919073.2
(22) Anmeldetag: 17.08.1993
(51) Int. Cl.: A61K 7/42, A61K 31/40, A61K 31/13, A61K 31/415, A61K 31/195, A61K 7/48

(54) **KOSMETISCHE UND DERMATOLOGISCHE LICHTSCHUTZFORMULIERUNGEN MIT EINEM GEHALT AN THIOLEN UND/ODER THIOLDERIVATEN**
COSMETIC AND DERMATOLOGICAL SUNSCREEN COMPOSITIONS CONTAINING THIOLS AND/OR THIOL DERIVATES
COMPOSITIONS COSMETIQUES ET DERMATOLOGIQUES ANTI-SOLAIRES CONTENANT DES THIOLS ET/OU DES DERIVES DE THIOL

(30) Priorität: 26.08.1992 DE 4228455
(43) Veröffentlichungstag der Anmeldung: 14.06.1995
(73) Patentinhaber: Beiersdorf Aktiengesellschaft, D-20245 Hamburg (DE); Rijksuniversiteit te Leiden, NL-2312 AV Leiden (NL)
(72) Erfinder: ALERT, Dirk, D-22607 Hamburg (DE); GERS-BARLAG, Heinrich, D-25495 Kummerfeld (DE); VAN DEN BROEKE, Leon T., NL-2334 EZ Leiden (NL); BEIJERSBERGEN VAN HENEGOUWEN, Gerard M.J., NL-2396 VL Koudekerk a/d Rijn (NL)
(86) Internationale Anmeldenummer: EP9302188
(87) Internationale Veröffentlichungsnummer: WO9404129

(56) Entgegenhaltungen:
- EP-A- 0 138 262
- EP-A- 0 170 048
- EP-A- 0 190 685
- EP-A- 0 219 455
- EP-A- 0 238 302
- EP-A- 0 356 119
- EP-A- 0 358 880
- EP-A- 0 500 332
- WO-A-91/16302
- WO-A-93/10755
- DE-A- 4 244 090
- FR-A- 2 340 932
- FR-A- 2 608 425
- FR-A- 2 666 226
- DATABASE WPI Week 7801, Derwent Publications Ltd., London, GB; AN 78-00904 & JP,A,52 136 925 (SANSEI SEIYAKU KK) 16. November 1977

## Beschreibung

Die vorliegende Erfindung betrifft Lichtschutzmittel, insbesondere kosmetische und dermatologische Lichtschutzmittel.

Die schädigende Wirkung des ultravioletten Teils der Sonnenstrahlung auf die Haut ist allgemein bekannt. Während Strahlen mit einer Wellenlänge, die kleiner als 290 nm ist (der sogenannte UVC-Bereich), von der Ozonschicht in der Erdatmosphäre absorbiert werden, verursachen Strahlen im Bereich zwischen 290 nm und 320 nm, dem sogenannten UVB-Bereich, ein Erythem, einen einfachen Sonnenbrand oder sogar mehr oder weniger starke Verbrennungen.

Als ein Maximum der Erythemwirksamkeit des Sonnenlichtes wird der engere Bereich um 308 nm angegeben.

Zum Schutz gegen UVB-Strahlung sind zahlreiche Verbindungen bekannt, bei denen es sich um Derivate des 3-Benzylidencamphers, der 4-Aminobenzoesäure, der Zimtsäure, der Salicylsäure, des Benzophenons sowie auch des 2-Phenylbenzimidazols handelt.

Auch für den Bereich zwischen etwa 320 nm und etwa 400 nm, den sogenannten UVA-Bereich, ist es wichtig, Filtersubstanzen zur Verfügung zu haben, da auch dessen Strahlen Schäden hervorrufen können. So ist erwiesen, daß UVA-Strahlung zu einer Schädigung der elastischen und kollagenen Fasern des Bindegewebes führt, was die Haut vorzeitig altem läßt, und daß sie als Ursache zahlreicher phototoxischer und photoallergischer Reaktionen zu sehen ist. Der schädigende Einfluß der UVB-Strahlung kann durch UVA-Strahlung verstärkt werden.

Zum Schutz gegen die Strahlen des UVA-Bereichs werden daher gewisse Derivate des Dibenzoylmethans verwendet, deren Photostabilität (Int. J. Cosm. Science 10, 53 (1988)) nicht in ausreichendem Maße gegeben ist.

Die UV-Strahlung kann aber auch zu photochemischen Reaktionen führen, wobei dann die photochemischen Reaktionsprodukte in den Hautmetabolismus eingreifen.

Vorwiegend handelt es sich bei solchen photochemischen Reaktionsprodukten um radikalische Verbindungen, beispielsweise Hydroxyradikale. Auch undefinierte radikalische Photoprodukte, welche in der Haut selbst entstehen, können aufgrund ihrer hohen Reaktivität unkontrollierte Folgereaktionen an den Tag legen. Aber auch Singulettsauerstoff, ein nichtradikalischer angeregter Zustand des Sauerstoffmoleküls kann bei UV-Bestrahlung auftreten, ebenso kurzlebige Epoxide und viele Andere. Singulettsauerstoff beispielsweise zeichnet sich gegenüber dem normalerweise vorliegenden Triplettsauerstoff (radikalischer Grundzustand) durch gesteigerte Reaktivität aus. Allerdings existieren auch angeregte, reaktive (radikalische) Triplettzustände des Sauerstoffmoleküls.

Um diesen Reaktionen vorzubeugen, können den kosmetischen bzw. dermatologischen Formulierungen zusätzlich Antioxidantien und/oder Radikalfänger einverleibt werden.

Die teilweise vorstehend genannten Verbindungen, welche als Lichtschutzmittel für kosmetische und dermatologische Lichtschutzformulierungen eingesetzt werden, zeichnen sich an sich durch gute Lichtschutzwirkung aus. Sie haben jedoch den Nachteil, daß es bisweilen schwierig ist, sie in befriedigender Weise solchen Formulierungen einzuverleiben. Ferner sind diese Verbindungen reine UV-Absorber und als Radikalfänger ungeeignet.

Es ist bereits vorgeschlagen worden, Vitamin E, eine Substanz mit bekannter antioxidativer Wirkung in Lichtschutzformulierungen einzusetzen, dennoch bleibt auch hier die erzielte Wirkung weit hinter der erhofften zurück.

Es war indes überraschend und für den Fachmann nicht vorauszusehen, daß die Verwendung von Thiolen und/oder Thiolderivaten zur Herstellung kosmetischer oder dermatologischer Lichtschutzformulierungen, wobei die Thiole und/oder Thiolderivate gewährt werden aus der Gruppe S-Acetylcystein, S-Acetylcysteinethylester, N-Acetylcysteinethylester, S,N-Diacetylcystein, S,N-Diacetylcysteinamid, wobei die Alkylreste der als Alkylester vorliegendenThiole bzw. Thiolderivate 1 bis 18 C-Atome aufweisen, und wobei die Thiole bzw. Thiolderivate gewünschtenfalls in Form ihrer kosmetisch oder pharmazeutisch akzeptablen Salze bzw. Säure- oder Basenaddukte vorliegen können den Nachteilen des Standes der Technik abhhilft.

Es war nicht vorherzusehen gewesen, daß die erfindungsgemäß verwendeten Thiole, bzw. Thiolderivate bzw. die diese enthaltenden kosmetischen oder dermatologischen Zubereitungen
- besser gegen Schädigung durch UV-Strahlung schützen
- besser als Antioxidans wirken
- besser als Radikalfänger wirken
- besser die Bindung von schädlichen Photoprodukten an Lipide, DNS und Proteine verhindern
würden als die Zubereitungen des Standes der Technik. Ferner war nicht vorherzusehen gewesen, daß die erfindungsgemäß verwendeten Thiole, bzw. Thiolderivate oder die erfindungsgemäßen kosmetischen oder dermatolgischen Zubereitungen
- für die Anwendung genügend hohe Stabilität aufweisen
- zu hautverträglichen Produkten führen
- nicht in die hauteigene Mikroorganismenflora eingreifen
- der lichtinduzierten Hautalterung entgegenwirken
würden.

Insbesondere war nicht vorherzusehen, daß die erfindungsgemäß verwendeten Thiole, bzw. Thiolderivate oder die erfindungsgemäßen kosmetischen oder dermatolgischen Zubereitungen sich durch eine ausgeprägte verzögerte Wirkung ("Retard-Wirkung") auszeichnen würden. Soll ein besonderes Gewicht auf diese Retard-Wirkung gelegt werden, so sind insbesondere die Thioester und Thioether bevorzugt.

Darüber hinaus sind die erfindungsgemäß verwendeten Thiole, bzw. Thiolderivate oder die erfindungsgemäßen kosmetischen oder dermatolgischen Zubereitungen in besonderem Maße zur Penetration in tiefergelegene Hautschichten geeignet, wo sie in vorteilhafter Weise ihre Wirkung entfalten können.

Weiterhin eignen sich die erfindungsgemäß verwendeten Thiole, bzw. Thiolderivate oder die erfindungsgemäßen kosmetischen oder dermatolgischen Zubereitungen erstaunlicherweise zur gezielten Prophylaxe und/oder Behandlung UV-induzierter Hautschäden.

Zwar waren aus EP-A 219 455 als Bräunungsmittel wirkende Zubereitungen bekannt, welche N-Acetylcystein enthalten. Insbesondere verweist diese Schrift darauf, daß N-Acetylcystein die Pigmentbildung fördere und hauptsächlich zu diesem Zwecke eingesetzt werde. Eine gewisse zusätzliche antierythematöse Wirkung, welche am angegebenen Orte beansprucht wird, mache es für kosmetische Zubereitungen geeignet. Die vorteilhaften Eigenschaften der vorliegenden Erfindung, nämlich die vorab geschilderten Vorteile, lehrt diese Schrift indes nicht.

In EP-A 138 262 wird eine Kombination aus Panthenol, Carrageenan und Verbindungen gewählt aus der Gruppe Methionin, Cystein, N-Acetylcystein, S-Acetylcystein, ferner anderen Bestandteilen. Diese Kombination dient der Behandlung von durch Sonnenlicht hervorgerufenem Ekzem und Dishydrosis. Auch durch diese Schrift wird keine Lehre vermittelt, welche in die Richtung der vorliegenden Erfindung weisen könnte.

Die erfindungsgemäß verwendeten Thiole bzw. Thiolderivate leiten sich vom Grundkörper Cystein wie folgt ab:

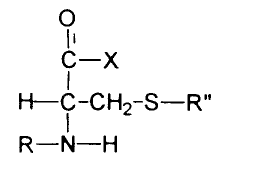

Der Rest X kann aus der Gruppe -O-R', -NRR' gewährt werden. Die Reste R, R' und R'' stellen unabhängig voneinander dar: H, C₁₋₁₈-Alkyl oder -Alkenyl, C₁₋₁₈-Acyl. Nicht von der vorstehenden Formel abgedeckt werden soll das N-Acetylcystein.

Bevorzugt ist dabei, die organischen Reste so zu wählen, daß die organischen Reste R, R' und R'' unanbhängig voneinander darstellen: H, Ethyl, Acetyl. X kann außerdem vorteilhaft die Gruppe -NH₂ symbolisieren.

Ferner sind von Vorteil die Salze bzw. Säure- oder Basenaddukte der erfindungsgemäßen Thiole bzw. Thiolderivate.

Beispiele für erfindungsgemäß besonders bevorzugte Thiole bzw. Thiolderivate sind

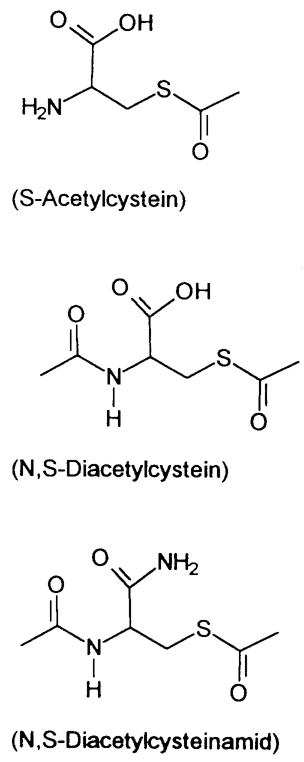

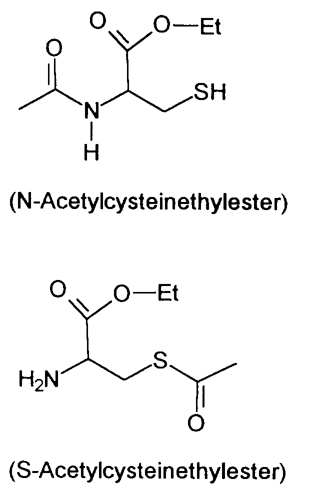

Bevorzugt sind auch die Salze bzw. Säure- oder Basenaddukte dieser besonders bevorzugten Thiole bzw. Thiolderivate.

Erfindungsgemäß ist demnach auch die Verwendung von Thiolen bzw. Thiolderivaten zum Schutze der Haut gegen schädlichen Einfluß von ultraviolettem Licht.

In erstaunlicher Weise hat sich herausgestellt, daß die erfindungsgemäßen Thiole bzw. Thiolderivate in der Lage sind, photochemisch erzeugte RadiKale abzufangen, vor photochemisch induzierten unkontrollierten Oxidationsprozessen zu schützen und sogar Singulettsauerstoff zu "quenchen", also durch einen physikochemischen Vorgang in den Triplettgrundzustand zu überführen. Stoffe mit dieser Eigenschaft werden auch als "Quenching Agents" bezeichnet.

Erfindungsgemäß ist daher auch die Verwendung von Thiolen bzw. Thiolderivaten als Radikalfänger, Antioxidans und/oder Quenching Agent für photochemisch erzeugte reaktive Substanzen wie Singulettsauerstoff.

Die erfindungsgemäßen kosmetischen und/oder dermatologischen Formulierungen können wie üblich zusammengesetzt sein und zur Behandlung der Haut und/oder der Haare im Sinne einer dermatologischen Behandlung oder einer Behandlung im Sinne der pflegenden Kosmetik dienen. Sie können aber auch in Schminkprodukten in der dekorativen Kosmetik eingesetzt werden. Sie enthalten bevorzugt 0,01 Gew.-% bis 10 Gew.-%, insbesondere aber 0,1 Gew.-% bis 6 Gew.-%, bezogen auf das Gesamtgewicht eines oder mehrerer Thiole bzw. Thiolderivate.

Zur Anwendung werden die erfindungsgemäßen Thiole bzw. Thiolderivate in der für Kosmetika und Dermatika üblichen Weise auf die Haut und/oder die Haare in ausreichender Menge aufgebracht.

Besonders bevorzugt sind solche kosmetische und dermatologische Zubereitungen, die in der Form eines Sonnenschutzmittels vorliegen. Vorteilhaft enthalten diese zusätzlich mindestens einen UVA-Filter und/oder mindestens einen UVB-Filter und/oder mindestens ein anorganisches Pigment.

Kosmetische Zubereitungen gemäß der Erfindung zum Schutze der Haut vor UV-Strahlen können in verschiedenen Formen vorliegen, wie sie z.B. üblicherweise für diesen Typ von Zubereitungen eingesetzt werden. So können sie z.B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W), ein Gel, eine Hydrodispersion, einen festen Stift oder auch ein Aerosol darstellen.

Die erfindungsgemäßen kosmetischen Zubereitungen können kosmetische Hilfsstoffe enthalten, wie sie üblicherweise in solchen Zubereitungen verwendet werden, z.B. Konservierungsmittel, Bakterizide, Parfüme, Mittel zum Verhindern des Schäumens, Farbstoffe, Pigmente, die eine färbende Wirkung haben, Verdikkungsmittel, oberflächenaktive Substanzen, Emulgatoren, weichmachende Substanzen, anfeuchtende und/oder feuchhaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Sofern die kosmetische oder dermatologische Zubereitung eine Lösung oder Lotion darstellt, können als Lösungsmittel verwendet werden:
- Wasser oder wäßrige Lösungen;
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Emulsionen gemäß der Erfindung z.B. in Form einer Sonnenschutzcreme, einer Sonnenschutzlotion oder einer Sonnenschutzmilch sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Feste Stifte gemäß der Erfindung enthalten z.B. natürliche oder synthetische Wachse, Fettalkohole oder Fettsäureester. Bevorzugt werden Lippenpflegestifte.

Als Treibmittel für erfindungsgemäße, aus Aerosolbehältern versprühbare kosmetische oder dermatologische Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Die kosmetischen und dermatologischen Zubereitungen zum Schutz der Haut enthalten die erfindungsgemäßen Thiole bzw. Thiolderivate z.B. in Mengen von 0,01 - 10 Gew.-%, vorzugsweise in Mengen von 0,1 - 6 Gew.-%, insbesondere aber 0,5 - 5 Gew.-% bezogen auf das Gesamtgewicht der Zubereitung.

Bevorzugt können die erfindungsgemäßen Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitung, um kosmetische Zubereitungen zur Verfügung zu stellen, die die Haut vor dem gesamten Bereich der ultravioletten Strahlung schützen. Sie können auch als Sonnenschutzmittel dienen.

Die UVB-Filter können öllöslich oder wasserlöslich sein. Als öllösliche Substanzen sind z.B. zu nennen:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsauredi(2-ethyhexyl)ester;
- 2,4,6-Trianilino-(p-carbo-2'-ethyl-1'-hexyloxy)-1,3,5-triazin.

Als wasserlösliche Substanzen sind z.B. zu nennen:
- Salze der 2-Phenylbenzimidazol-5-sulfonsäure wie ihr Natrium-, Kaum- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Die Liste der genannten UVB-Filter, die in Kombination mit den erfindungsgemäßen Thiolen bzw. Thiolderivaten verwendet werden können, soll selbstverständlich nicht limitierend sein.

Gegenstand der Erfindung ist auch die Kombination eines oder mehrerer erfindungsgemäßer Thiole bzw. Thiolderivate mit einem oder mehreren UVB-Filtern bzw. erfindungsgemäße kosmetische oder dermatologische Zubereitungen, welches auch einen oder mehrere UVB-Filter enthalten.

Es kann auch von Vorteil sein, ein oder mehrere erfindungsgemäße Thiole bzw. Thiolderivate mit UVA-Filtern zu kombinieren, die bisher üblicherweise in kosmetischen und/oder dermatologischen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch diese Kombinationen bzw. Zubereitungen, die diese Kombinationen enthalten, sind Gegenstand der Erfindung. Es können die für die UVB-Kombination verwendeten Mengen eingesetzt werden.

Ferner werden vorteilhafte Zubereitungen erhalten, wenn die erfindungsgemäßen Thiole bzw. Thiolderivate mit UVA- und UVB-Filtern kombiniert werden.

Kosmetische Zubereitungen, Thiole enthaltend, können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agentien sind. Besonders bevorzugt handelt es sich um Pigmente auf der Basis von Titandioxid.

Auch diese Kombinationen von UVA-Filter und/oder UVB-Filter und Pigment bzw. Zubereitungen, die diese Kombination enthalten, sind Gegenstand der Erfindung. Es können die für die vorstehenden Kombinationen genannten Mengen verwendet werden.

Bei kosmetischen Zubereitungen zum Schutz der Haare vor UV-Strahlen gemäß der Erfindung handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisier- und Behandlungslotion, einen Haarlack oder um Dauerwellmittel. Die kosmetischen Zubereitungen enthalten Wirkstoffe und Hilfsstoffe, wie sie üblicherweise für diesen Typ von Zubereitungen zur Haarpflege und Haarbehandlung verwendet werden. Als Hilfsstoffe dienen Konservierungsmittel, oberflächenaktive Substanzen, Substanzen zum Verhindern des Schäumens, Emulgatoren, Verdickungsmittel, Fette, Öle, Wachse, organische Lösungsmittel, Bakterizide, Parfüme, Farbstoffe oder Pigmente, deren Aufgabe es ist, die Haare oder die Zubereitung selbst zu färben, Elektrolyte, Zubereitungen gegen das Fetten der Haare.

Kosmetische Zubereitungen, die ein Shampoonierungsmittel darstellen, enthalten vorzugsweise mindestens eine anionische, nicht-ionische oder amphotere oberflächenaktive Substanz oder Gemische daraus, mindestens ein erfindungsgemäßes Thiol bzw. Thiolderivat im wäßrigen Medium und Hilfsmittel, wie sie üblicherweise dafür verwendet werden. Die oberflächenaktive Substanz kann in einer Konzentration zwischen 1 Gew.-% und 50 Gew.-% in dem Shampoonierungsmittel vorliegen.

Liegt die kosmetische oder dermatologische Zubereitung in Form einer Lotion vor, die ausgespült und z.B. vor oder nach der Entfärbung, vor oder nach der Shampoonierung, zwischen zwei Shampoonierungsschritten, vor oder nach der Dauerwellbehandlung angewendet wird, so handelt es sich dabei z.B. um wäßrige oder wäßrig-alkoholische Lösungen, die gegebenenfalls oberflächenaktive Substanzen enthalten, bevorzugt nichtionische oder kationische oberflächenaktive Substanzen, deren Konzentration zwischen 0,1 und 10 Gew.-%, vorzugsweise zwischen 0,2 und 5 Gew.-%, liegen kann. Diese kosmetische oder dermatologische Zubereitung kann auch ein Aerosol mit den üblicherweise dafür verwendeten Hilfsmitteln darstellen.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine wäßrige, alkoholische oder wäßrig-alkoholische Lösung dar und enthält mindestens ein kationisches, anionisches, nicht-ionisches oder amphoteres Polymer oder auch Gemische derselben, sowie mindestens ein erfindungsgemäßes Thiol bzw. Thiolderivat. Die Menge der verwendeten Polymeren liegt z.B. zwischen 0,1 und 10 Gew.-%, bevorzugt zwischen 0,1 und 3 Gew.-%.

Kosmetische und dermatologische Zubereitungen zur Behandlung und Pflege der Haare, die mindestens ein erfindungsgemäßes Thiol bzw. Thiolderivat enthalten, können als Emulsionen vorliegen, die vom nicht-ionischen oder anionischen Typ sind. Nicht-ionische Emulsionen enthalten neben Wasser Öle oder Fettalkohole, die beispielsweise auch polyethoxyliert oder polypropoxyliert sein können, oder auch Gemische aus den beiden organischen Komponenten. Diese Emulsionen enthalten gegebenenfalls kationische oberflächenaktive Substanzen. Anionische Emulsionen sind vorzugsweise vorn Typ einer Seife und enthalten mindestens ein erfindungsgemäßes Thiol bzw. Thiolderivat mit anionischem oder nicht-ionischem Charakter.

Kosmetische und dermatologische Zubereitungen zur Behandlung und Pflege der Haare können als Gele vorliegen, die neben mindestens einem erfindungsgemäßes Thiol bzw. Thiolderivat und dafür üblicherweise verwendeten Lösungsmitteln noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Vorzugsweise beträgt die Menge der erfindungsgemäßen Thiole bzw. Thiolderivate in einem für die Haare bestimmten Mittel 0,01 Gew.-% bis 10 Gew.%, insbesondere 0,5 Gew.-% bis 5 Gew.-%, bezogen auf das Gesamtgewicht der Zubereitungen.

Die vorliegende Erfindung umfaßt auch ein Verfahren zum Schutze der Haut und der Haare vor UVA- und UVB-Strahlung, das dadurch gekennzeichnet ist, daß man eine kosmetische oder dermatologische Zubereitung, welche mindestens ein erfindungsgemäßes Thiol bzw. Thiolderivat enthält, in ausreichender Menge auf die Haut oder Haare aufbringt, sowie die Verwendung dieser Verbindungen insbesondere für diese Zwecke.

Ebenso umfaßt die vorliegende Erfindung auch ein Verfahren zum Schutz farbloser oder gefärbter kosmetischer und dermatologischer Zubereitungen gegen UVA- und UVB-Strahlen sowie diese Zubereitungen, bei denen es sich z.B. um vorstehend genannte Zubereitungen zur Behandlung und Pflege der Haare, insbesondere um Haarfärbemittel, Haarlacke, Shampoonierungsmittel, Farbshampoonierungsmittel, um Schminkprodukte wie z.B. Nagellacke, Lippenstifte, Teintgrundlagen, Cremes zur Behandlung der Haut oder um sämtliche anderen kosmetischen Zubereitungen handelt, deren Bestandteile Stabilitätsprobleme aufgrund von Licht bei der Lagerung mit sich bringen können, dadurch gekennzeichnet, daß den kosmetischen oder dermatologischen Zubereitungen mindestens ein erfindungsgemäßes Thiol bzw. Thiolderivat in für die Stabilisierung gegenüber Licht, insbesondere UVA-strahlen, ausreichender Menge zugesetzt wird.

Vorzugsweise beträgt die Menge der erfindungsgemäßen Verbindungen in diesen Zubereitungen 0,01 Gew.-% bis 10 Gew.-%, insbesondere 0,1 Gew.-% bis 3 Gew.-%, bezogen auf das Gesamtgewicht der Zubereitungen.

Alle Mengenangaben, Anteile und Prozentanteile sind, soweit nicht anders angegeben, auf das Gewicht und die Gesamtmenge bzw. auf das Gesamtgewicht der Zubereitungen bezogen.

Gegenstand der Erfindung ist auch das Verfahren zur Herstellung der erfindungsgemäßen kosmetischen Zubereitungen, das dadurch gekennzeichnet ist, daß man in an sich bekannter Weise erfindungsgemäße Thiole bzw. Thiolderivate in kosmetische oder dermatologische Formulierungen einarbeitet.

Die nachfolgenden Beispiele sollen die vorliegende Erfindung verdeutlichen, ohne sie einzuschränken.

| Beispiel 1 | |
|---|---|
| | Gew.-% |
| Cyclomethicone | 2,000 |
| Cetyldimethicone Copolyol | 0,200 |
| PEG-22-Dodecyl Copolymer | 3,000 |
| Paraffinöl (DAB 9) | 2,000 |
| Caprylsäure/Caprinsäure Triglycerid | 5,800 |
| Octylmethoxycinnamat | 5,800 |
| Butyl-methoxy-dibenzoylmethan | 4,000 |
| Verbindung I | 0,300 |
| ZnSO₄ | 0,700 |
| Na₄EDTA | 0,300 |
| Parfum, Konservierungsmittel, Farbstoffe | nach Belieben |
| H₂O VES | ad 100,000 |

| Beispiel 2 | |
|---|---|
| | Gew.-% |
| Cyclomethicone | 2,000 |
| Cetyldimethicone Copolyol | 0,200 |
| PEG-22-Dodecyl Copolymer | 3,000 |
| Paraffinöl (DAB 9) | 2,000 |
| Caprylsäure-/Caprinsäure Triglycerid | 5,800 |
| Octylmethoxycinnamat | 5,800 |
| Butyl-methoxy-dibenzoylmethan | 4,000 |
| Verbindung II | 0,500 |
| ZnSO₄ | 0,700 |
| Na₄EDTA | 0,300 |
| Parfum, Konservierungsmittel, Farbstoffe | nach Belieben |
| H₂O VES | ad 100,000 |

| Beispiel 3 | |
|---|---|
| | Gew.-% |
| Cyclomethicone | 2,000 |
| Cetyldimethicone Copolyol | 0,200 |
| PEG-22-Dodecyl Copolymer | 3,000 |
| Paraffinöl (DAB 9) | 2,000 |
| Caprylsäure-/Caprinsäure Triglycerid | 5,800 |
| Octylmethoxycinnamat | 5,800 |
| Butyl-methoxy-dibenzoylmethan | 4,000 |
| Verbindung III | 2,500 |
| ZnSO₄ | 0,700 |
| Na₄EDTA | 0,300 |
| Parfum, Konservierungsmittel, Farbstoffe | nach Belieben |
| H₂O VES | ad 100,000 |

| Beispiel 4 | |
|---|---|
| | Gew.-% |
| Cyclomethicone | 2,000 |
| Cetyldimethicone Copolyol | 0,200 |
| PEG-22-Dodecyl Copolymer | 3,000 |
| Paraffinöl (DAB 9) | 2,000 |
| Caprylsäure-/Caprinsäure Triglycerid | 5,800 |
| Octylmethoxycinnamat | 5,800 |
| Butyl-methoxy-dibenzoylmethan | 4,000 |
| Verbindung IV | 3,000 |
| ZnSO₄ | 0,700 |
| Na₄EDTA | 0,300 |
| Parfum, Konservierungsmittel, Farbstoffe | nach Belieben |
| H₂O VES | ad 100,000 |

| Beispiel 5 | |
|---|---|
| Cyclomethicone | 2,000 |
| Cetearylalkohol + PEG-40-hydriertes Rizinusöl + Natrium Cetearylsulfat | 2,500 |
| Glyceryllanolat | 1,000 |
| Caprylsäure-/Caprinsäure Triglycerid | 0,100 |
| Laurylmethicon Copolyol | 2,000 |
| Octylstearat | 3,000 |
| Rizinusöl | 4,000 |
| Glycerin | 3,000 |
| Acrylamid/Natriumacrylat Copolymer | 0,300 |
| Hydroxypropylmethylcellulose | 0,300 |
| Octylmethoxycinnamat | 5,000 |
| Butyl-methoxy-dibenzoylmethan | 0,500 |
| Verbindung V | 1,750 |
| Na_{3HEDTA} | 1,500 |
| Parfum, Konservierungsmittel, Farbstoffe | nach Belieben |
| H₂O VES | ad 100,000 |

| Beispiel 6 | |
|---|---|
| Cyclomethicone | 2,000 |
| Cetearylalkohol + PEG-40-hydriertes Rizinusöl + Natrium Cetearylsulfat | 2,500 |
| Glyceryllanolat | 1,000 |
| Caprylsäure-/Caprinsäure Triglycerid | 0,100 |
| Laurylmethicon Copolyol | 2,000 |
| Octylstearat | 3,000 |
| Rizinusöl | 4,000 |
| Glycerin | 3,000 |
| Acrylamid/Natriumacrylat Copolymer | 0,300 |
| Hydroxypropylmethylcellulose | 0,300 |
| Octylmethoxycinnamat | 5,000 |
| Butyl-methoxy-dibenzoylmethan | 0,750 |
| Verbindung VI | 2,500 |
| Na_{3HEDTA} | 1,500 |
| Parfum, Konservierungsmittel, Farbstoffe | nach Belieben |
| H₂O VES | ad 100,000 |

| Beispiel 7 | |
|---|---|
| Cyclomethicone | 2,000 |
| Cetearylalkohol + PEG-40-hydriertes Rizinusöl + Natrium Cetearylsulfat | 2,500 |
| Glyceryllanolat | 1,000 |
| Caprylsäure-/Caprinsäure Triglycerid | 0,100 |
| Laurylmethicon Copolyol | 2,000 |
| Octylstearat | 3,000 |
| Rizinusöl | 4,000 |
| Glycerin | 3,000 |
| Acrylamid/Natriumacrylat Copolymer | 0,300 |
| Hydroxypropylmethylcellulose | 0,300 |
| Octylmethoxycinnamat | 5,000 |
| Butyl-methoxy-dibenzoylmethan | 1,000 |
| Verbindung VII | 0,700 |
| Na_{3HEDTA} | 1,500 |
| Parfum, Konservierungsmittel, Farbstoffe | nach Belieben |
| H₂O VES | ad 100,000 |

| Beispiel 8 | |
|---|---|
| Cyclomethicone | 2,000 |
| Cetearylalkohol + PEG-40-hydriertes Rizinusöl + Natrium Cetearylsulfat | 2,500 |
| Glyceryllanolat | 1,000 |
| Caprylsäure-/Caprinsäure Triglycerid | 0,100 |
| Laurylmethicon Copolyol | 2,000 |
| Octylstearat | 3,000 |
| Rizinusöl | 4,000 |
| Glycerin | 3,000 |
| Acrylamid/Natriumacrylat Copolymer | 0,300 |
| Hydroxypropylmethylcellulose | 0,300 |
| Octylmethoxycinnamat | 5,000 |
| Butyl-methoxy-dibenzoylmethan | 0,500 |
| Verbindung VIII | 1,600 |
| Na_{3HEDTA} | 1,500 |
| Parfum, Konservierungsmittel, Farbstoffe | nach Belieben |
| H₂O VES | ad 100,000 |

## Patentansprüche

1. Verwendung von Thiolen und/oder Thiolderivaten zur Herstellung kosmetischer oder dermatologischer Lichtschutzformulierungen, wobei die Thiole und/oder Thiolderivate gewählt werden aus der Gruppe S-Acetylcystein, S-Acetylcystein-alkylester, N-Acetylcysteinalkylester, S,N-Diacetylcystein, S,N-Diacetylcysteinalkylester, wobei die Alkylreste der als Alkylester vorliegenden Thiole bzw. Thiolderivate 1 bs 18 C-Atome aufweisen, und wobei die Thiole bzw. Thiolderivate gewünschtenfalls in Form ihrer kosmetisch oder pharmazeutisch akzeptablen Salze bzw. Säure- oder Basenaddukte vorliegen können.

2. Verwendung nach Anspruch 1, dadurch gekennzeichnet, daß die Alkylreste der als Alkylester vorliegenden Thiole bzw. Thiolderivate Ethylreste darstellen.

3. Verwendung nach Anspruch 1, dadurch gekennzeichnet, daß die Thiole bzw. Thiolderivate in Kombination mit UV-Licht absorbierenden Substanzen vorliegen.

4. Verwendung nach Anspruch 1, dadurch gekennzeichnet, daß die Lichtschutzformulierungen einen Gehalt von 0,01 - 10 Gew.-%, vorzugsweise von 0,1 - 6 Gew.-%, insbesondere 0,5 - 5 Gew.-% an den erfindungsgemäßen Thiolen bzw. Thiolderivaten aufweisen, jeweils bezogen auf das Gesamtgewicht der Zubereitungen.

## Claims

1. Use of thiols and/or thiol derivatives for the preparation of cosmetic or dermatological light protection formulations, in which the thiols and/or thiol derivatives are chosen from the group consisting of S-acetylcysteine, S-acetylcysteine alkyl esters, N-acetylcysteine alkyl esters, S,N-diacetylcysteine, S,N-diacetylcysteine alkyl esters, the alkyl radicals of the thiols or thiol derivatives which are present as alkyl esters having 1 to 18 C atoms and the thiols or thiol derivatives being able to be present, if desired, in the form of their cosmetically or pharmaceutically acceptable salts or acid or base adducts.

2. Use according to Claim 1, characterized in that the alkyl radicals of the thiols or thiol derivatives which are present as alkyl esters represent ethyl radicals.

3. Use according to Claim 1, characterized in that the thiols or thiol derivatives are present in combination with substances which absorb UV light.

4. Use according to Claim 1, characterized in that the light protection formulations have a content of 0.01 - 10% by weight, preferably 0.1 - 6% by weight, in particular 0.5 - 5% by weight, of the thiols or thiol derivatives according to the invention, in each case based on the total weight of the formulations.

## Revendications

1. Utilisation de thiols et/ou de dérivés de thiols pour la préparation de compositions cosmétiques ou dermatologiques de protection contre la lumière, les thiols et/ou dérivés de thiols étant choisis parmi la S-acétylcystéine, un ester alkylique de S-acétylcystéine, un ester alkylique de N-acétylcystéine, la S,N-diacétylcystéine, un ester alkylique de S,N-diacétylcystéine, les radicaux alkyle des thiols ou dérivés de thiols se trouvant sous forme d'esters alkyliques ayant de 1 à 18 atomes de carbone, et les thiols ou dérivés de thiols pouvant si on le désire se trouver sous forme de leurs sels ou produits d'addition avec des acides ou des bases, cosmétiquement ou pharmaceutiquement acceptables.

2. Utilisation selon la revendication 1, caractérisée en ce que les radicaux alkyle des thiols ou dérivés de thiols se trouvant sous forme d'esters alkyliques représentent des radicaux éthyle.

3. Utilisation selon la revendication 1, caractérisée en ce que les thiols ou dérivés de thiols se trouvent en association avec des substances absorbant la lumière UV.

4. Utilisation selon la revendication 1, caractérisée en ce que les compositions de protection contre la lumière ont une teneur en thiols ou dérivés de thiols selon l'invention allant de 0,01 à 10 % en poids, de préférence de 0,1 à 6 % en poids, en particulier de 0,5 à 5 % en poids, dans chaque cas par rapport au poids total des compositions.
